# EUROPEAN PATENT SPECIFICATION

(11) **EP 2 932 887 B1**
(45) Date of publication and mention of the grant of the patent: **06.09.2017**
(21) Application number: 13835074.9
(22) Date of filing: 23.07.2013
(51) Int. Cl.: A61B 1/07

(54) **ANTI-FOG ENDOSCOPE SYSTEM DEVICE AND METHOD**
BESCHLAGHEMMENDE VORRICHTUNG UND VERFAHREN FÜR ENDOSKOPSYSTEM
SYSTÈME D'ENDOSCOPE ANTIBUÉE, DISPOSITIF ET PROCÉDÉ

(30) Priority: 05.09.2012 CN 201210324982
(43) Date of publication of application: 21.10.2015
(73) Proprietor: Qingdao Novelbeam Technology Co. Ltd., Shandong 266101 (CN)
(72) Inventor: ZHENG, Yao, Qingdaoshi Shandong 266101 (CN); GU, Changming, Qingdaoshi Shandong 266101 (CN); ZHENG, Anmin, Qingdaoshi Shandong 266101 (CN); MAO, Rongzhuang, Qingdaoshi Shandong 266101 (CN); YANG, Chunxin, Qingdaoshi Shandong 266101 (CN)
(74) Representative: Cabinet Chaillot
(86) International application number: PCT/CN2013/079898
(87) International publication number: WO 2014/036861

(56) References cited:
- CN-A- 102 292 014
- JP-A- 2003 334 157
- US-A- 4 279 246
- US-A- 5 605 532
- US-A- 5 647 840
- US-B1- 6 503 196

## Description

### Technical field of the invention

The invention relates to a device of anti-fogging endoscope system and its method, falling in the minimally invasive medical technical field.

### Background of the invention

Medical endoscopes have been widely used in the minimally invasive surgery, however, the current minimally invasive surgery using an endoscope system has an operating deficiency, i.e., when an endoscope is working in a human body, due to a temperature difference between the internal and external of the human body, a part of the water vapor which contacts the endoscope having a lower temperature usually forms fog on a distal optical window plate of the endoscope to influence the imaging clarity of the surgical field, which blurs the image of surgical field showing on a displayer, and affects the observing and operating of a doctor. In order to remove the fogging on the endoscope, doctors mainly apply ways of using physiological saline to preheat endoscopes, smearing anti-fogging oil and so on, which have some effect on relieving the problem of fogging on the endoscope, while their defect is making the surgical preparation complex, there is a latent risk of incompletely disinfecting, and the time of surgical preparation is prolonged. Accordingly, the best way is directly providing an anti-fogging or defogging solution on an endoscope system. For that purpose, document JP 2003 334157 A discloses in essence an endoscope with a white light source and a separate infrared light source both being coupled to the distal observation window by individual light guides.

### Summary of the invention

The invention aims to solve a technical problem of providing a device of anti-fogging endoscope system and its method, wherein a near-infrared lighting source for anti-fogging is added on the basis of the traditional endoscope system, a beam of which is coupled into the lighting transmission path in color combination, and changes material properties of a distal optical window plate to transmit the visible light, ensuring that surgical filed is lighted by white light, while the temperature of the distal optical window plate is elevated by absorbing energy of the near-infrared light to reduce the temperature difference between the distal optical window plate of endoscope and a human body, to realize the purpose of anti-fogging.

Now, combining with the accompanying figure, the technical solution of the invention is specified:
A device of anti-fogging endoscope system, including a lighting source 1, a transmission fiber 2, and an endoscope 3, wherein the lighting source 1 includes a lighting source of white light 11 and a coupling optical lens 14; the lighting source of white light 11 is a xenon lamp, a white light LED or a mixed white light LED of three primary colors of red, green, and blue, which provides a light output in visible light band; when the xenon lamp is used, the power falls in the range of 250W∼350W; when the white light LED is used, the power falls in the range of 50W∼100W; when the mixed white light LED of three primary colors of red, green, and blue is used, the power falls in the range of 150W∼200W; the coupling optical lens 14 is an optical lens or a set of optical lenses; the transmission fiber 2 is made by binding several fibers having the diameter of several tens of microns together, and is polished on its incident surface and exit surface; the focusing plane of coupling optical lens 14 and the incident surface of transmission fiber 2 are in coincidence; the endoscope 3 includes an endoscope optical lens system 31, a distal optical window plate 32, a light guide fiber bundle 33, an endoscope housing body 34, and a fiber connecting interface 35; the endoscope optical lens system 31 is made of several tens of optical lenses to image the surgical field; the endoscope housing body 34 is mainly made from stainless steel material, which is polished on its surface; the distal optical window plate 32 is a transparent optical material, and can transmit the visible light, which is located in front of the endoscope optical lens system 31, and is welded or adhered to the endoscope housing body 34; the light guide fiber bundle 33 is made by binding several fibers having the diameter of several tens of microns together, and is polished on its incident surface and exit surface; the incident surface of light guide fiber bundle 33 is located inside the fiber connecting interface 35; the incident surface of light guide fiber bundle 33 is connected to the exit surface of transmission fiber 2 in coupling via the fiber connecting interface 35; the light guide fiber bundle 33 is located in the annular space between the optical lens system 31 and the endoscope housing body 34, of which exit surface is against the distal optical window plate 32, which characterized in that, the lighting source 1 also includes a near-infrared lighting source for anti-fogging 12 and a color combination filter 13; the color combination filter 13 is a optical plate, which is located between the lighting source of white light 11 and the coupling optical lens 14 in way of having a intersection angle of 45° from the coupling optical lens 14; the color combination filter 13 is plated with an optical film which transmits the visible light and reflects the near-infrared light; the near-infrared lighting source for anti-fogging 12 is a light source which only emits the near-infrared light, such as a light source of semiconductor laser, a light source of pump laser, and so on, of which the wavelength range of the emitted light is 780nm∼1100nm, and the power of the emitted light in full power is between 5 watt and 10 watt; the near-infrared lighting source for anti-fogging 12 is set in way of being perpendicular to the lighting source of white light 11 and having a intersection angle of 45° from the color combination filter 13; the lighting source of white light 11 and the near-infrared lighting source for anti-fogging 12 can be independently controlled to turn on-off and adjust luminance; the distal optical window plate 32 of the endoscope 3 is made from an optical material improving its spectral characteristics, which not only can transmit the visible light, but also can absorb the near-infrared light, wherein the average transmissivity of the visible light is above 80%, and the average absorptivity of the near-infrared light is above 80%.

According to the above device of anti-fogging endoscope system, an anti-fogging method of the anti-fogging endoscope system including the following steps:
(1) 2∼3 minutes before an operation, the near-infrared lighting source for anti-fogging 12 is turned on in full power, near-infrared light transmits through the coupling optical lens 14, the transmission fiber 2, and the light guide fiber bundle 33 in order, and then irradiates on the distal optical window plate 32; the distal optical window plate 32 absorbs energy of the near-infrared light, and the temperature rises up.
(2) After 30 seconds ∼ 1minute, the temperature of the distal optical window plate 32 rises from room temperature of 25 up to about 37°C, then the power of the near-infrared lighting source for anti-fogging 12 is reduced to 10%∼30% of full power to keep the temperature of the distal optical window plate 32 around 37°C.
(3) Before the doctor inserts the endoscope into a human body, the near-infrared lighting source for anti-fogging 12 is turned off, the lighting source of white light 11 is turned on, and then the endoscope is inserted into the human body; due to the temperature of the distal optical window plate 32 is approximately consistent with the temperature of the human body, no fogging will happen, and so the doctor can perform the normal observation and operation, to achieve the purpose of anti-fogging.

The invention has the following positive improvements: no great change will be made on the basis of the traditional endoscope system, only a near-infrared lighting source for anti-fogging is added, and the material of the distal optical window plate is changed to achieve an anti-fogging function on system level. Comparing with anti-fogging ways of preheating with physiological saline, smearing anti-fogging oil, and so on, as to the doctor's performances, the invention has the more convenient and easier advantages, and there is not a latent risk of incompletely disinfecting when using physiological saline, anti-fogging oil and so on.

### Brief description of the drawings

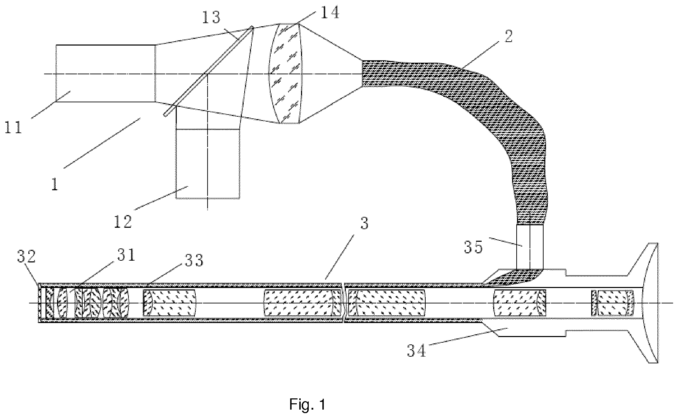

Fig. 1 is a structural schematic diagram of a device of anti-fogging endoscope system disclosed in the invention.

### Detailed description of the invention

Now, the technical solution and working principle of the invention are specified by combining with the accompanying figure and embodiments.

All the embodiments have completely same structures and methods with that of the device and method described in the summary of the invention. In order to avoid a repetition, the following embodiments only present critical technical data.

### Embodiment 1

The lighting source of white light 11 is a white light LED having a total power of 80W. The near-infrared lighting source for anti-fogging 12 is a semiconductor laser having a radiation wavelength of 808 nm and a full power output of 5W. The distal optical window plate 32 has an absorptivity of 80% at 808 nm, and the average transmissivity of the visible light is 90%. The near-infrared lighting source for anti-fogging 12 is turned on in full power last 1 minute, the temperature of the distal optical window plate 32 rises from room temperature of 25°C up to about 37°C, then the power is reduced to 30% of full power, keeping the temperature around 37°C.

The working principle is: 2∼3 minutes before an operation, the semiconductor laser of 808 nm is turned on to output a laser of 808 nm with 5W. The laser of 808 nm passes through the color combination filter 13, and then is reflected into the coupling optical lens 14. The coupling optical lens 14 makes the near-infrared light of 808 nm focus and couple into the transmission fiber 2, and exit from the exit surface of the transmission fiber 2. The exited near-infrared light of 808 nm couples into the light guide fiber bundle 33 via the fiber connecting interface 35, and then exit from the exit surface of the light guide fiber bundle 33. The exited near-infrared light of 808 nm irradiates onto the distal optical window plate 32, the near-infrared light of 80% is absorbed by the distal optical window plate, and then the temperature of the distal optical window plate 32 begins to rise up from the room temperature. After 1 minute, the temperature of distal optical window plate rises up to around 37°C. At this time, the power of the near-infrared lighting source for anti-fogging 12 is reduced to 30% of full power to keep the temperature of distal optical window plate around 37°C. Before the endoscope 3 is inserted into a human body, the near-infrared lighting source for anti-fogging 12 is turned off. Then the lighting source of white light 11 is turned on, and the white light emitted from the lighting source of white light 11 irradiates to the color combination filter 13, and passes through the color combination filter 13 into the coupling optical lens 14. The coupling optical lens 14 makes the white light focus and couple into the transmission fiber 2, and exit from the exit surface of the transmission fiber 2. The exited white light couples into the light guide fiber bundle 33 via the fiber connecting interface 35, and then exit from the exit surface of the light guide fiber bundle 33. The exited white light irradiates onto the distal optical window plate 32, and white light of about 90% transmits through the distal optical window plate 32 to light the surgical field. In this way, due to the temperature of the distal optical window plate 32 is approximately consistent with the temperature of the human body inside, no fogging will happen on the distal optical window plate 32, while the while light lights the surgical field to ensure the normal observation and operation performances in the surgical field.

### Embodiment 2:

The lighting source of white light 11 is a mixed white light LED of three primary colors of red, green, and blue having a total power of 170W. The near-infrared lighting source for anti-fogging 12 is a semiconductor laser having a radiation wavelength of 940 nm and a full power output of 10W. The distal optical window plate 32 has an absorptivity of 90% at 940 nm, and the average transmissivity of the visible light is 85%. The near-infrared lighting source for anti-fogging 12 is turned on in full power last 30 seconds, the temperature of the distal optical window plate 32 rises from room temperature of 25°C up to about 37°C, then the power is reduced to 10% of full power, keeping the temperature around 37°C.

### Embodiment 3:

The lighting source of white light 11 is a xenon lamp having a total power of 350W. The near-infrared lighting source for anti-fogging 12 is a Nd:YAG laser having a radiation wavelength of 1064 nm and a full power output of 7W. The distal optical window plate 32 has an absorptivity of 95% at 1064 nm, and the average transmissivity of the visible light is 80%. The near-infrared lighting source for anti-fogging 12 is turned on in full power last 45 seconds, the temperature of the distal optical window plate 32 rises from room temperature of 25°C up to about 37°C, then the power is reduced to 20% of full power, keeping the temperature around 37°C.

The working principles of embodies 2 and 3 is similar to that of embody 1, and so there is unnecessary to repeat.

The device of anti-fogging endoscope system and its method disclosed in the invention is suitable to be used in various endoscopic operations, and is specially suitable to be used in the endoscopic operations wherein the endoscope needs to be repeatedly inserted into the human body and drawn out from the human body, in order to play the effect of anti-fogging.

## Claims

1. A device of anti-fogging endoscope system, including a lighting source (1), a transmission fiber (2), and an endoscope (3), wherein the lighting source (1) includes a lighting source of white light (11) and a coupling optical lens (14); the lighting source of white light (11) is a xenon lamp, a white light LED or a mixed white light LED of three primary colors of red, green, and blue, which provides a light output in visible light band; when the xenon lamp is used, the power falls in the range of 250W∼350W; when the white light LED is used, the power falls in the range of 50W∼100W; when the mixed white light LED of three primary colors of red, green, and blue is used, the power falls in the range of 150W∼200W; the coupling optical lens (14) is an optical lens or a set of optical lenses; the transmission fiber (2) is made by binding several fibers having the diameter of several tens of microns together, and is polished on its incident surface and exit surface; the focusing plane of coupling optical lens (14) and the incident surface of transmission fiber (2) are in coincidence; the endoscope (3) includes an endoscope optical lens system (31), a distal optical window plate (32), a light guide fiber bundle (33), an endoscope housing body (34), and a fiber connecting interface (35); the endoscope optical lens system (31) is made of several tens of optical lenses to image the surgical field; the endoscope housing body (34) is mainly made from stainless steel material, which is polished on its surface; the distal optical window plate (32) is a transparent optical material, and can transmit the visible light, which is located in front of the endoscope optical lens system (31), and is welded or adhered to the endoscope housing body (34); the light guide fiber bundle (33) is made by binding several fibers having the diameter of several tens of microns together, and is polished on its incident surface and exit surface; the incident surface of light guide fiber bundle (33) is located inside the fiber connecting interface (35); the incident surface of light guide fiber bundle (33) is connected to the exit surface of transmission fiber (2) in coupling via the fiber connecting interface (35); the light guide fiber bundle (33) is located in the annular space between the optical lens system (31) and the endoscope housing body (34), of which exit surface is against the distal optical window plate (32), wherein the lighting source (1) also includes a near-infrared lighting source for anti-fogging (12) and a color combination filter (13); the color combination filter (13) is a optical plate, which is located between the lighting source of white light (11) and the coupling optical lens (14) in way of having a intersection angle of 45° from the coupling optical lens (14); the color combination filter (13) is plated with an optical film which transmits the visible light and reflects the near-infrared light; the near-infrared lighting source for anti-fogging (12) is a light source which only emits the near-infrared light, of which the wavelength range of the emitted light is 780nm∼1100nm, and the power of the emitted light in full power is between 5 watt and 10 watt; the near-infrared lighting source for anti-fogging (12) is set in way of being perpendicular to the lighting source of white light (11) and having a intersection angle of 45° from the color combination filter (13); the lighting source of white light (11) and the near-infrared lighting source for anti-fogging (12) can be independently controlled to turn on-off and adjust luminance; the distal optical window plate (32) of the endoscope (3) is made from an optical material improving its spectral characteristics, which not only can transmit the visible light, but also can absorb the near-infrared light, wherein the average transmissivity of the visible light is above 80%, and the average absorptivity of the near-infrared light is above 80%.

2. The device of anti-fogging endoscope system according to claim 1, which **characterized in that**, the lighting source of white light (11) is a white light LED having a total power of 80W; the near-infrared lighting source for anti-fogging (12) is a semiconductor laser having a radiation wavelength of 808 nm and a full power output of 5W; the distal optical window plate (32) has an absorptivity of 80% at 808 nm, and the average transmissivity of the visible light is 90%; the near-infrared lighting source for anti-fogging (12) is turned on in full power last 1 minute, the temperature of the distal optical window plate (32) rises from room temperature of 25°C up to about 37°C, then the power is reduced to 30% of full power, keeping the temperature around 37°C.

3. The device of anti-fogging endoscope system according to claim 1, which **characterized in that**, the lighting source of white light (11) is a mixed white light LED of three primary colors of red, green, and blue having a total power of 170W; the near-infrared lighting source for anti-fogging (12) is a semiconductor laser having a radiation wavelength of 940 nm and a full power output of 10W; the distal optical window plate (32) has an absorptivity of 90% at 940 nm, and the average transmissivity of the visible light is 85%; the near-infrared lighting source for anti-fogging (12) is turned on in full power last 30 seconds, the temperature of the distal optical window plate (32) rises from room temperature of 25°C up to about 37°C, then the power is reduced to 10% of full power, keeping the temperature around 37°C.

4. The device of anti-fogging endoscope system according to claim 1, which **characterized in that**, the lighting source of white light (11) is a xenon lamp having a total power of 350W; the near-infrared lighting source for anti-fogging (12) is a Nd:YAG laser having a radiation wavelength of 1064 nm and a full power output of 7W; the distal optical window plate (32) has an absorptivity of 95% at 1064 nm, and the average transmissivity of the visible light is 80%; the near-infrared lighting source for anti-fogging (12) is turned on in full power last 45 seconds, the temperature of the distal optical window plate (32) rises from room temperature of 25°C up to about 37°C, then the power is reduced to 20% of full power, keeping the temperature around 37°C.

5. An anti-fogging method, performed by the device of anti-fogging endoscope system according to one of claims 1 to 4, including the following steps:
(1) 2∼3 minutes before an operation, the near-infrared lighting source for anti-fogging (12) is turned on in full power, near-infrared light transmits through the coupling optical lens (14), the transmission fiber (2), and the light guide fiber bundle (33) in order, and then irradiates on the distal optical window plate (32); the distal optical window plate (32) absorbs energy of the near-infrared light, and the temperature rises up;
(2) After 30 seconds ∼1minute, the temperature of the distal optical window plate rises from room temperature of 25°C up to about 37°C, then the power of the near-infrared lighting source for anti-fogging (12) is reduced to 10%∼30% of full power to keep the temperature of the distal optical window plate around 37°C; before the endoscope is inserted into the human body the near-infrared lightning source for anti-fogging is turned off and due to the temperature of the distal optical window plate (32) being approximately consistent with the temperature of the human body, no fogging will happen.

## Patentansprüche

1. Beschlaghemmende Vorrichtung für Endoskopsystem einschließlich einer Lichtquelle (1), einer Übertragungsfaser (2) und eines Endoskops (3), wobei die Lichtquelle (1) eine Lichtquelle von weißem Licht (11) und eine optische Koppellinse (14) umfasst; die Lichtquelle von weißem Licht (11) ist eine Xenonlampe, eine Weißlicht-LED oder eine vermischte Weißlicht-LED von drei Primärfarben rot, grün und blau, die einen Lichtausstoß im sichtbaren Lichtband liefert; wenn die Xenonlampe benutzt wird, liegt die Energie im Bereich von 250W∼350W; wenn die Weißlicht-LED benutzt wird, liegt die Energie im Bereich von 50W∼100W; wenn die vermischte Weißlicht-LED von drei Primärfarben rot, grün, und blau benutzt wird, liegt die Energie im Bereich von 150W∼200W; die optische Koppellinse (14) ist eine optische Linse oder ein Satz von optischen Linsen; die Übertragungsfaser (2) wird hergestellt, indem man mehrere Fasern mit dem Durchmesser von mehreren Dutzend Mikron zusammenbindet, und ist auf ihrer Einfallfläche und Austrittsfläche poliert; die Fokusebene der optischen Koppellinse (14) und die Einfallfläche der Übertragungsfaser (2) stimmen überein; das Endoskop (3) umfasst ein optisches Linsensystem für Endoskop (31), eine distale optische Fensterplatte (32), ein Lichtleiterfaserbündel (33), einen Endoskopgehäusekörper (34) und eine Faserverbindungsschnittstelle (35); das optische Linsensystem für Endoskop (31) besteht aus mehreren Dutzend optischer Linsen zur Abbildung des chirurgischen Feldes; der Endoskopgehäusekörper (34) ist vorwiegend aus Edelstahlmaterial, das auf seiner Oberfläche poliert ist; die distale optische Fensterplatte (32) ist ein transparentes optisches Material und kann das sichtbare Licht übertragen, das sich vor dem optischen Linsensystem für Endoskop (31) befindet, und ist an den Endoskopgehäusekörper (34) geschweißt oder liegt an ihn an; das Lichtleiterfaserbündel (33) wird hergestellt, indem man mehrere Fasern mit dem Durchmesser von mehreren Dutzend Mikron zusammenbindet, und ist auf seiner Einfall- und Austrittsfläche poliert; die Einfallfläche des Lichtleiterfaserbündels (33) befindet sich in der Faserverbindungsschnittstelle (35); die Einfallfläche des Lichtleiterfaserbündels (33) ist mit der Austrittsfläche der Übertragungsfaser (2) in Kupplung mittels der Faserverbindungsschnittstelle (35) verbunden; das Lichtleiterfaserbündel (33) befindet sich in dem ringförmigen Raum zwischen dem optischen Linsensystem (31) und dem Endoskopgehäusekörper (34), dessen Austrittsfläche gegen die distale optische Fensterplatte (32) ist, wobei die Lichtquelle (1) auch eine Nahinfrarotlichtquelle zur Beschlaghemmung (12) und einen Farbenkombinationsfilter (13) umfasst; der Farbenkombinationsfilter (13) ist eine optische Platte, die sich zwischen der Lichtquelle von weißem Licht (11) und der optischen Koppellinse (14) befindet, so dass ein Schnittpunktwinkel von 45° von der optischen Koppellinse (14) entsteht; der Farbenkombinationsfilter (13) ist mit einem optischen Film überzogen, der das sichtbare Licht überträgt und das Nahinfrarotlicht reflektiert; die Nahinfrarotlichtquelle zur Beschlaghemmung (12) ist eine Lichtquelle, die nur das Nahinfrarotlicht ausstrahlt, dessen Wellenlängenbereich des ausgestrahlten Lichts 780nm∼1100nm ist, und die Energie des ausgestrahlten Lichts bei voller Leistung liegt zwischen 5 Watt und 10 Watt; die Nahinfrarotlichtquelle zur Beschlaghemmung (12) liegt senkrecht zu der Lichtquelle von weißem Licht (11) und umfasst einen Schnittpunktwinkel von 45° von dem Farbenkombinationsfilter (13); die Lichtquelle von weißem Licht (11) und die Nahinfrarotlichtquelle zur Beschlaghemmung (12) können unabhängig voneinander zum Ein- und Ausschalten und Regeln der Leuchtkraft gesteuert werden; die distale optische Fensterplatte (32) des Endoskops (3) ist aus einem optischen Material, das ihre spektralen Eigenschaften verbessert, die nicht nur das sichtbare Licht übertragen können, sondern auch das Nahinfrarotlicht absorbieren können, wobei die durchschnittliche Transmissivität des sichtbaren Lichtes über 80% beträgt und das durchschnittliche Absorptionsvermögen für Nahinfrarotlicht über 80%.

2. Beschlaghemmende Vorrichtung für Endoskopsystem nach Anspruch 1, **gekennzeichnet dadurch dass** die Lichtquelle von weißem Licht (11) eine Weißlicht-LED mit einer Gesamtleistung von 80W ist; die Nahinfrarotlichtquelle zur Beschlaghemmung (12) ist ein Halbleiterlaser mit einer Strahlungswellenlänge von 808 nm und einer vollen Leistung von 5W; die distale optische Fensterplatte (32) hat ein Absorptionsvermögen von 80% bei 808 nm und die durchschnittliche Transmissivität des sichtbaren Lichtes beträgt 90%; die Nahinfrarotbeleuchtungsquelle zur Beschlaghemmung (12) wird in der letzten 1 Minute auf volle Leistung geschaltet, die Temperatur der distalen optische Fensterplatte (32) steigt von Raumtemperatur 25°C auf etwa 37°C, dann wird die Energie auf 30% der vollen Leistung verringert und die Temperatur auf 37°C gehalten.

3. Beschlaghemmende Vorrichtung für Endoskopsystem nach Anspruch 1, **gekennzeichnet dadurch, dass** die Lichtquelle von weißem Licht (11) eine vermischte Weißlicht-LED von drei Primärfarben rot, grün und blau mit einer Gesamtleistung von 170W ist; die Nahinfrarotbeleuchtungsquelle zur Beschlaghemmung (12) ist ein Halbleiterlaser mit einer Strahlungswellenlänge von 940 nm und einem vollen Leistung von 10W; die distale optische Fensterplatte (32) hat ein Absorptionsvermögen von 90% bei 940 nm, und die durchschnittliche Transmissivität des sichtbaren Lichtes beträgt 85%; die Nahinfrarotlichtquelle zur Beschlaghemmung (12) wird in den letzten 30 Sekunden auf volle Leistung geschaltet, die Temperatur der distalen optische Fensterplatte (32) steigt von Raumtemperatur 25°C auf etwa 37°C, dann wird die Energie auf 10% der vollen Leistung verringert und die Temperatur auf 37°C gehalten.

4. Beschlaghemmende Vorrichtung für Endoskopsystem nach Anspruch 1, **gekennzeichnet dadurch, dass** die Lichtquelle von weißem Licht (11) eine Xenonlampe mit einer Gesamtleistung von 350W ist; die Nahinfrarotlichtquelle zur Beschlaghemmung (12) ist ein Nd:YAG-Laser mit einer Strahlungswellenlänge von 1064 nm und einer vollen Leistung von 7W; die distale optische Fensterplatte (32) hat ein Absorptionsvermögen von 95% bei 1064 nm, und die durchschnittliche Transmissivität des sichtbaren Lichtes beträgt 80%; die Nahinfrarotlichtquelle zur Beschlaghemmung (12) wird in den letzten 45 Sekunden auf volle Leistung geschaltet, die Temperatur der distalen optische Fensterplatte (32) steigt von Raumtemperatur 25°C auf etwa 37°C, dann wird die Energie auf 20% der vollen Leistung verringert und die Temperatur auf 37°C gehalten.

5. Beschlaghemmendes Verfahren, durchgeführt von der beschlaghemmenden Vorrichtung für Endoskopsystem nach einem der Ansprüche 1 bis 4, mit folgenden Phasen:
(1) 2∼3 Minuten vor einer Operation wird die Nahinfrarotlichtquelle zur Beschlaghemmung (12) auf volle Leistung geschaltet, Nahinfrarotlicht, überträgt durch die optische Koppellinse (14) die Übertragungsfaser (2), und das Lichtleiterfaserbündel (33) in dieser Reihenfolge, und bestrahlt dann die distale optische Fensterplatte (32); die distale optische Fensterplatte (32) absorbiert Energie des Nahinfrarotlichts, und die Temperatur steigt;
(2) Nach 30 Sekunden ∼ 1 Minute steigt die Temperatur der distalen optischen Fensterplatte von Raumtemperatur 25°C auf etwa 37°C, dann wird die Energie der Nahinfrarotlichtquelle zur Beschlaghemmung (12) auf 10%∼30% der vollen Leistung verringert, um die Temperatur der distalen optische Fensterplatte auf 37°C zu halten; bevor das Endoskop in den menschlichen Körper eingeführt wird, wird die Nahinfrarotlichtquelle zur Beschlaghemmung abgeschaltet und aufgrund der Temperatur der distalen optische Fensterplatte (32) ist diese zirka in Einklang mit der Temperatur des menschlichen Körpers, und kein Beschlagen wird auftreten.

## Revendications

1. Dispositif de système d'endoscope antibuée, comprenant une source d'éclairage (1), une fibre de transmission (2) et un endoscope (3), la source d'éclairage (1) comprenant une source d'éclairage de lumière blanche (11) et une lentille optique de couplage (14) ; la source d'éclairage de lumière blanche (11) est une lampe à xénon, une DEL à lumière blanche ou une DEL à lumière blanche mélangée de trois couleurs primaires de rouge, vert et bleu, qui fournit une sortie de lumière dans une bande de lumière visible ; lorsque la lampe à xénon est utilisée, la puissance appartient à la plage de 250 W-350 W ; lorsque la DEL à lumière blanche est utilisée, la puissance appartient à la plage de 50 W-100 W ; lorsque la DEL à lumière blanche mélangée de trois couleurs primaires de rouge, vert et bleu est utilisée, la puissance appartient à la plage de 150 W-200 W ; la lentille optique de couplage (14) est une lentille optique ou un ensemble de lentilles optiques ; la fibre de transmission (2) est réalisée en reliant ensemble plusieurs fibres ayant un diamètre de plusieurs dizaines de microns, et est polie sur sa surface d'incidence et sa surface de sortie ; le plan de focalisation de la lentille optique de couplage (14) et la surface d'incidence de la fibre de transmission (2) sont en coïncidence ; l'endoscope (3) comprend un système de lentille optique d'endoscope (31), une plaque de fenêtre optique distale (32), un faisceau de fibres de guidage de lumière (33), un corps de boîtier d'endoscope (34) et une interface de liaison de fibre (35) ; le système de lentille optique endoscope (31) est fait de plusieurs dizaines de lentilles optiques pour représenter une image du champ chirurgical ; le corps de boîtier d'endoscope (34) est principalement fait de matériau en acier inoxydable, qui est poli sur sa surface ; la plaque de fenêtre optique distale (32) est un matériau optique transparent, et peut transmettre la lumière visible, qui est situé devant le système de lentille optique d'endoscope (31), et est soudé ou collé sur le corps de boîtier d'endoscope (34) ; le faisceau de fibres de guidage de lumière (33) est réalisé en reliant ensemble plusieurs fibres ayant un diamètre de plusieurs dizaines de microns, et est poli sur sa surface d'incidence et sa surface de sortie ; la surface d'incidence du faisceau de fibres de guidage de lumière (33) est située à l'intérieur de l'interface de liaison de fibre (35) ; la surface d'incidence du faisceau de fibres de guidage de lumière (33) est reliée à la surface de sortie de la fibre de transmission (2) en couplage par l'intermédiaire de l'interface de liaison de fibre (35) ; le faisceau de fibres de guidage de lumière (33) est situé dans l'espace annulaire entre le système de lentille optique (31) et le corps de boîtier d'endoscope (34), dont la surface de sortie est contre la plaque de fenêtre optique distale (32), la source d'éclairage (1) comprenant également une source d'éclairage dans le proche infrarouge pour antibuée (12) et un filtre de combinaison de couleurs (13) ; le filtre de combinaison de couleurs (13) est une plaque optique, laquelle est située entre la source d'éclairage de lumière blanche (11) et la lentille optique de couplage (14) de manière à avoir un angle d'intersection de 45° par rapport à la lentille optique de couplage (14) ; le filtre de combinaison de couleurs (13) est plaqué d'un film optique qui transmet la lumière visible et réfléchit la lumière dans le proche infrarouge ; la source d'éclairage dans le proche infrarouge pour antibuée (12) est une source de lumière qui émet uniquement la lumière dans le proche infrarouge, dont la gamme de longueur d'onde de la lumière émise est 780 nm∼1100 nm, et la puissance de la lumière émise à pleine puissance est comprise entre 5 watts et 10 watts ; la source d'éclairage dans le proche infrarouge pour antibuée (12) est installée de manière à être perpendiculaire à la source d'éclairage de lumière blanche (11) et à avoir un angle d'intersection de 45° par rapport au filtre de combinaison de couleurs (13) ; la source d'éclairage de lumière blanche (11) et la source d'éclairage dans le proche infrarouge pour antibuée (12) peuvent être commandées de manière indépendante pour être allumées-éteintes et ajuster la luminance ; la plaque de fenêtre optique distale (32) de l'endoscope (3) est faite d'un matériau optique améliorant ses caractéristiques spectrales, qui peut non seulement transmettre la lumière visible, mais encore peut absorber la lumière dans le proche infrarouge, la transmissivité moyenne de la lumière visible étant supérieure à 80 %, et l'absorptivité moyenne de la lumière dans le proche infrarouge étant supérieure à 80 %.

2. Dispositif de système d'endoscope antibuée selon la revendication 1, **caractérisé par le fait que** la source d'éclairage de lumière blanche (11) est une DEL à lumière blanche ayant une puissance totale de 80 W ; la source d'éclairage dans le proche infrarouge pour antibuée (12) est un laser à semi-conducteur ayant une longueur d'onde de rayonnement de 808 nm et une sortie à pleine puissance de 5 W ; la plaque de fenêtre optique distale (32) a une absorptivité de 80 % à 808 nm, et la transmissivité moyenne de la lumière visible est de 90 % ; la source d'éclairage dans le proche infrarouge pour antibuée (12) est allumée à pleine puissance pendant 1 minute, la température de la plaque de fenêtre optique distale (32) augmente de la température ambiante de 25°C jusqu'à environ 37°C, puis la puissance est réduite à 30 % de la pleine puissance, maintenant la température autour de 37°C.

3. Dispositif de système d'endoscope antibuée selon la revendication 1, **caractérisé par le fait que** la source d'éclairage de lumière blanche (11) est une DEL à lumière blanche mélangée de trois couleurs primaires de rouge, vert et bleu ayant une puissance totale de 170 W ; la source d'éclairage dans le proche infrarouge pour antibuée (12) est un laser à semi-conducteur ayant une longueur d'onde de rayonnement de 940 nm et une sortie à pleine puissance de 10 W ; la plaque de fenêtre optique distale (32) a une absorptivité de 90 % à 940 nm, et la transmissivité moyenne de la lumière visible est de 85 % ; la source d'éclairage dans le proche infrarouge pour antibuée (12) est allumée à pleine puissance pendant 30 secondes, la température de la plaque de fenêtre optique distale (32) augmente de la température ambiante de 25°C jusqu'à environ 37°C, puis la puissance est réduite à 10 % de la pleine puissance, maintenant la température autour de 37°C.

4. Dispositif de système d'endoscope antibuée selon la revendication 1, **caractérisé par le fait que** la source d'éclairage de lumière blanche (11) est une lampe à xénon ayant une puissance totale de 350 W ; la source d'éclairage dans le proche infrarouge pour antibuée (12) est un laser Nd:YAG ayant une longueur d'onde de rayonnement de 1064 nm et une sortie à pleine puissance de 7 W ; la plaque de fenêtre optique distale (32) a une absorptivité de 95 % à 1064 nm, et la transmissivité moyenne de la lumière visible est de 80 % ; la source d'éclairage dans le proche infrarouge pour antibuée (12) est allumée à pleine puissance pendant 45 secondes, la température de la plaque de fenêtre optique distale (32) augmente de la température ambiante de 25°C jusqu'à environ 37°C, puis la puissance est réduite à 20 % de la pleine puissance, maintenant la température autour de 37°C.

5. Procédé antibuée mis en oeuvre par le dispositif de système d'endoscope antibuée selon l'une des revendications 1 à 4, comprenant les étapes suivantes :
(1) 2∼3 minutes avant un fonctionnement, la source d'éclairage dans le proche infrarouge pour antibuée (12) est allumée à pleine puissance, la lumière dans le proche infrarouge est transmise à travers la lentille optique de couplage (14), la fibre de transmission (2) et le faisceau de fibres de guidage de lumière (33) dans cet ordre, puis irradie la plaque de fenêtre optique distale (32) ; la plaque de fenêtre optique distale (32) absorbe de l'énergie de la lumière proche infrarouge, et la température augmente ;
(2) après 30 secondes∼1 minute, la température de la plaque de fenêtre optique distale augmente de la température ambiante de 25°C jusqu'à environ 37°C, puis la puissance de la source d'éclairage dans le proche infrarouge pour antibuée (12) est réduite à 10 %-30 % de la pleine puissance pour maintenir la température de la plaque de fenêtre optique distale autour de 37°C ; avant l'introduction de l'endoscope dans le corps humain, la source d'éclairage dans le proche infrarouge pour antibuée est éteinte et, en raison de la température de la plaque de fenêtre optique distale (32) approximativement égale à la température du corps humain, aucune buée n'apparaît.
